# EUROPEAN PATENT APPLICATION

(11) **EP 4 778 448 A1**
(43) Date of publication of application: **22.07.2026**
(21) Application number: 24865727.2
(22) Date of filing: 28.08.2024
(51) Int. Cl.: A61B 5/00, A61B 5/318, G16H 50/20, G16H 50/50, G16H 10/60, G06N 3/08

(54) **METHOD FOR GENERATING DISEASE PREDICTION INFORMATION USING BIOMETRIC SIGNAL**

(30) Priority: 14.09.2023 KR 20230122607
(71) Applicant: VUNO Inc., Seoul 06541 (KR)
(72) Inventor: LEE, Sungjae, 06541 Seoul (KR); JOO, Sunghoon, 04778 Seoul (KR)
(74) Representative: Buzzi, Notaro & Antonielli d'Oulx S.p.A.
(86) International application number: PCT/KR2024/012826
(87) International publication number: WO 2025/058286

(57) **Abstract**

A method for generating disease prediction information using a biological signal, performed by a computing device according to an embodiment of the present disclosure, is disclosed. The method may include obtaining a biological signal at a time point of diagnosis, obtaining a past biological signal at a past time point prior to the time point of diagnosis or information associated with the past biological signal, and generating disease prediction information for the time point of diagnosis based on the biological signal at the time point of diagnosis and the past biological signal at the past time point or the information associated with the past biological signal using a neural network model.

## Description

### [Technical Field]

The present invention relates to a method for generating disease prediction information, and more particularly, to a technique for generating disease prediction information for a time point of diagnosis using a biological signal at the time point of diagnosis and additional information.

### [Background Art]

With the development of medical measurement technology, an individual can visit a medical institution such as a hospital and easily and inexpensively measure a biological signal, and with the development of information and communication technology, the measured biological signal can be easily managed through databasing and the like.

In addition, recently, through a portable measurement device or a wearable device, an individual can obtain various biological signals relatively easily and inexpensively even without visiting a medical institution, and a history of the obtained biological signals can be easily managed through various data management systems.

Meanwhile, in most cases, an artificial intelligence model for diagnosing a specific disease predicts a probability of occurrence of the disease based on a single biological signal measured at a time point of diagnosis.

In other words, in most cases, the artificial intelligence model predicts a disease based on a biological signal of a single time point (for example, a biological signal at a current time point of diagnosis).

Therefore, the artificial intelligence model could not comprehensively consider biological signals at various time points or consider a history of the biological signals, and since the artificial intelligence model predicts a disease based only on a fragmentary biological signal of a single time point, there is a problem in that prediction accuracy is relatively low.

Korean Registered Patent No. 10-0748184 (Aug. 03, 2007) discloses an electrocardiogram-based cardiac disease diagnosis apparatus and method using a neural network.

### [Disclosure]

### [Technical Problem]

The present disclosure aims to provide a technique for more accurately predicting disease prediction information for a time point of diagnosis by using a past biological signal at a past time point in addition to a biological signal at the time point of diagnosis.

Meanwhile, the technical object to be achieved by the present disclosure is not limited to the above-mentioned technical object, and various technical objects may be included within the scope apparent to those skilled in the art from the following description.

### [Technical Solution]

According to an embodiment of the present disclosure for achieving the above-described technical object, a method for generating disease prediction information using a biological signal performed by a computing device is disclosed. The method may comprise obtaining a biological signal at a time point of diagnosis; obtaining a past biological signal at a past time point prior to the time point of diagnosis or information associated with the past biological signal; and generating disease prediction information for the time point of diagnosis based on the biological signal at the time point of diagnosis and the past biological signal at the past time point or the information associated with the past biological signal, using a neural network model.

In an embodiment, generating the disease prediction information for the time point of diagnosis may comprise generating a first vector based on the biological signal; generating a second vector based on the past biological signal or the information associated with the past biological signal; and generating the disease prediction information for the time point of diagnosis based on the first vector and the second vector, using the neural network model.

In an embodiment, the information associated with the past biological signal may comprise at least one of time difference information between the past time point and the time point of diagnosis; medical information at the past time point; or reference prediction information generated for the past biological signal using a reference neural network model different from the neural network model.

In an embodiment, the medical information at the past time point may comprise medical measurement information measured within a predetermined time from an obtained time of the past biological signal.

In an embodiment, the reference neural network model may be a model configured to generate a reference prediction value based on a biological signal of a single time point.

In an embodiment, generating the disease prediction information for the time point of diagnosis based on the first vector and the second vector, using the neural network model may comprise generating an integrated vector based on the first vector and the second vector; and generating the disease prediction information for the time point of diagnosis based on the integrated vector, using the neural network model.

In an embodiment, the first vector and the second vector may be vectors generated through different pre-processing methods, the first vector and the second vector may comprise different numbers of elements, and the integrated vector may comprise more elements of the first vector than elements of the second vector.

In an embodiment, obtaining the past biological signal at the past time point prior to the time point of diagnosis or the information associated with the past biological signal may comprise obtaining a plurality of past biological signals for a plurality of past time points prior to the time point of diagnosis or information associated with the plurality of past biological signals, and generating the second vector based on the past biological signal or the information associated with the past biological signal may comprise generating the second vector based on information associated with the plurality of past biological signals.

In an embodiment, the information associated with the plurality of past biological signals may comprise respective individual information associated with each past biological signal included in the plurality of past biological signals, and the respective individual information associated with each past biological signal may comprise at least one of time difference information between the respective past time point and the time point of diagnosis; medical information mapped to the respective past biological signal; or reference prediction information generated for the respective past biological signal using a reference neural network model different from the neural network model.

In an embodiment, generating the second vector based on information associated with the plurality of past biological signals may comprise generating supplementary biological signals in a number corresponding to a difference between the predetermined number and the number of the plurality of past biological signalsusing at least one past biological signal among the plurality of past biological signals when the number of the plurality of past biological signals is less than a predetermined number; and generating the second vector based on the plurality of past biological signals and information associated with the supplementary biological signals.

In an embodiment, the supplementary biological signals may be generated based on a past biological signal of the earliest past time point among the plurality of past biological signals.

In an embodiment, the information associated with the plurality of past biological signals may comprise tabular data generated based on the plurality of past biological signals, and the tabular data may comprise at least one type of data among a first type of data related to time difference; a second type of data related to medical information; a third type of data related to disease prediction based on a single time point; or a fourth type of data related to missing information.

According to an embodiment of the present disclosure for achieving the above-described technical object, a structure of a neural network model implemented by a computing device is disclosed. The structure of the neural network model may comprise a preprocessing module configured to preprocess a biological signal at a time point of diagnosis and a past biological signal at a past time point prior to the time point of diagnosis or information associated with the past biological signal; and a prediction module configured to generate disease prediction information based on data related to the biological signal generated by the preprocessing module and data related to the past biological signal generated by the preprocessing module.

In an embodiment, the preprocessing module may comprise a first encoder module configured to generate a first vector based on the biological signal at the time point of diagnosis; a second encoder module configured to generate a second vector based on the past biological signal or the information associated with the past biological signal; and an integration module configured to generate an integrated vector based on the first vector and the second vector.

In an embodiment, the prediction module may generate the disease prediction information based on the integrated vector.

According to an embodiment of the present disclosure for achieving the above-described technical object, a computer program stored in a non-transitory computer-readable storage medium is disclosed. When executed on one or more processors, the computer program causes the one or more processors to perform operations for generating disease prediction information using a biological signal, the operations comprising: an operation of obtaining a biological signal at a time point of diagnosis; an operation of obtaining a past biological signal at a past time point prior to the time point of diagnosis or information associated with the past biological signal; and an operation of generating disease prediction information for the time point of diagnosis based on the biological signal at the time point of diagnosis and the past biological signal at the past time point or the information associated with the past biological signal, using a neural network model.

According to an embodiment of the present disclosure for achieving the above-described technical object, a computing device is disclosed. The computing device may comprise at least one processor and memory, and the at least one processor may be configured to obtain a biological signal at a time point of diagnosis; obtain a past biological signal at a past time point prior to the time point of diagnosis or information associated with the past biological signal; and generate disease prediction information for the time point of diagnosis based on the biological signal at the time point of diagnosis and the past biological signal at the past time point or the information associated with the past biological signal, using a neural network model.

### [Advantageous Effects]

The present disclosure can further improve accuracy of disease prediction of the neural network model by additionally using a past biological signal at a past time point in addition to a biological signal at a time point of diagnosis. For example, the present disclosure can implement a neural network model in which a complex correlation among the biological signal at the time point of diagnosis, the past biological signal at the past time point, and a disease is learned, and accuracy of disease prediction can be further improved by using such a neural network model.

In addition, the present disclosure can further improve accuracy of disease prediction by using a neural network model that considers various types of information associated with the past biological signal at the past time point. For example, the present disclosure may use various secondary processed information associated with the past biological signal as inputs of the neural network model so that the neural network model learns various correlations associated with the past biological signal, and accuracy of disease prediction can be further improved through such operations.

In addition, the present disclosure can further improve accuracy of disease prediction by using a neural network model that additionally considers information regarding a plurality of past biological signals at a plurality of past time points in addition to the biological signal at the time point of diagnosis. For example, the present disclosure can implement a neural network model in which correlations related to a history of biological signals are more deeply reflected, and accuracy of disease prediction can be further improved through such a neural network model.

Meanwhile, effects of the present disclosure are not limited to the effects described above, and various effects may be included within a scope apparent to those skilled in the art from the following description.

### [Description of Drawings]

FIG. 1 is a block diagram of a computing device for generating disease prediction information using a biological signal according to an embodiment of the present disclosure.
FIG. 2 is a conceptual diagram illustrating a neural network according to an embodiment of the present disclosure.
FIG. 3 is a flowchart illustrating a method for generating disease prediction information using a biological signal according to an embodiment of the present disclosure.
FIG. 4 is a diagram for explaining an embodiment of information associated with a single past biological signal according to an embodiment of the present disclosure.
FIG. 5 is a diagram for explaining an operation of generating reference prediction information for a past biological signal using a reference neural network model different from the neural network model according to an embodiment of the present disclosure.
FIG. 6 is a diagram for explaining information associated with a plurality of past biological signals according to an embodiment of the present disclosure.
FIG. 7 is a diagram for explaining an operation of generating a second vector when the number of the plurality of past biological signals satisfies a predetermined number according to an embodiment of the present disclosure.
FIG. 8 is a diagram for explaining an operation of generating a second vector when the number of the plurality of past biological signals is less than a predetermined number according to an embodiment of the present disclosure.
FIG. 9 is a block diagram of a structure of a neural network model for generating disease prediction information using a biological signal according to an embodiment of the present disclosure.
FIG. 10 is a diagram schematically illustrating an operation of generating disease prediction information in the neural network model according to an embodiment of the present disclosure.
FIG. 11 is a normal and schematic view of an exemplary computing environment in which the exemplary embodiments of the present disclosure may be implemented.

### [Mode for Invention]

Various exemplary embodiments will now be described with reference to drawings. In the present specification, various descriptions are presented to provide appreciation of the present disclosure. However, it is apparent that the exemplary embodiments can be executed without the specific description.

"Component", "module", "system", and the like which are terms used in the specification refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally In terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented exemplary embodiments is provided so that those skilled in the art of the present disclosure use or implement the present disclosure. Various modifications to the exemplary embodiments will be apparent to those skilled in the art. Generic principles defined herein may be applied to other embodiments without departing from the scope of the present disclosure. Therefore, the present disclosure is not limited to the exemplary embodiments presented herein. The present disclosure should be analyzed within the widest range which is coherent with the principles and new features presented herein.

In the present disclosure, the network function and the artificial neural network and the neural network may be used interchangeably.

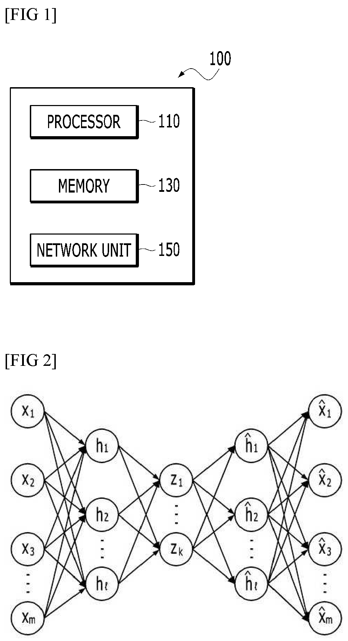

FIG. 1 is a block diagram of a computing device for generating disease prediction information using a biological signal according to an embodiment of the present disclosure.

A configuration of the computing device 100 illustrated in FIG. 1 is only an example shown through simplification. In an exemplary embodiment of the present disclosure, the computing device 100 may include other components for performing a computing environment of the computing device 100 and only some of the disclosed components may constitute the computing device 100.

The computing device 100 may include a processor 110, a memory 130, and a network unit 150.

The processor 110 may be constituted by one or more cores and may include processors for data analysis and deep learning, which include a central processing unit (CPU), a general purpose graphics processing unit (GPGPU), a tensor processing unit (TPU), and the like of the computing device. The processor 110 may read a computer program stored in the memory 130 to perform data processing for machine learning according to an exemplary embodiment of the present disclosure. According to an exemplary embodiment of the present disclosure, the processor 110 may perform a calculation for training the neural network. At least one of the CPU, GPGPU, and TPU of the processor 110 may process training of a network function. For example, both the CPU and the GPGPU may process the training of the network function and data classification using the network function. Further, in an exemplary embodiment of the present disclosure, processors of a plurality of computing devices may be used together to process the training of the network function and the data classification using the network function. Further, the computer program executed in the computing device according to an exemplary embodiment of the present disclosure may be a CPU, GPGPU, or TPU executable program.

According to one embodiment of the present disclosure, the memory 130 can store any form of information generated or determined by the processor 110 and any form of information received by the network unit 150.

According to an exemplary embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

The network unit 150 according to an exemplary embodiment of the present disclosure may use various wired communication systems such as public switched telephone network (PSTN), x digital subscriber line (xDSL), rate adaptive DSL (RADSL), multi rate DSL (MDSL), very high speed DSL (VDSL), universal asymmetric DSL (UADSL), high bit rate DSL (HDSL), and local area network (LAN).

The network unit 150 presented in the present disclosure may use various wireless communication systems such as code division multi access (CDMA), time division multi access (TDMA), frequency division multi access (FDMA), orthogonal frequency division multi access (OFDMA), single carrier-FDMA (SC-FDMA), and other systems.

In the present disclosure, the network unit 150 may be configured regardless of the communication mode, such as wired or wireless, and may be configured as various communication networks, such as a local area network (LAN), a personal area network (PAN), and a wide area network (WAN). In addition, the network may be the well-known World Wide Web (WWW), and may also utilize a wireless transmission technology used for short-distance communication, such as infrared (IrDA: Infrared Data Association) or Bluetooth.

The techniques described in the present disclosure may also be used in other networks mentioned above.

FIG. 2 is a conceptual diagram illustrating a neural network according to an embodiment of the present disclosure.

Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, a definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

In the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, generative adversarial networks (GAN), a restricted Boltzmann machine (RBM), a deep belief network (DBN), a Q network, a U network, a Siam network, a Generative Adversarial Network (GAN), and the like. The description of the deep neural network described above is just an example and the present disclosure is not limited thereto.

In an exemplary embodiment of the present disclosure, the network function may include the auto encoder. The auto encoder may be a kind of artificial neural network for outputting output data similar to input data. The auto encoder may include at least one hidden layer and odd hidden layers may be disposed between the input and output layers. The number of nodes in each layer may be reduced from the number of nodes in the input layer to an intermediate layer called a bottleneck layer (encoding), and then expanded symmetrical to reduction to the output layer (symmetrical to the input layer) in the bottleneck layer. The auto encoder may perform non-linear dimensional reduction. The number of input and output layers may correspond to a dimension after preprocessing the input data. The auto encoder structure may have a structure in which the number of nodes in the hidden layer included in the encoder decreases as a distance from the input layer increases. When the number of nodes in the bottleneck layer (a layer having a smallest number of nodes positioned between an encoder and a decoder) is too small, a sufficient amount of information may not be delivered, and as a result, the number of nodes in the bottleneck layer may be maintained to be a specific number or more (e.g., half of the input layers or more).

The neural network may be trained in at least one scheme of supervised learning, unsupervised learning, semi supervised learning, or reinforcement learning. The learning of the neural network may be a process in which the neural network applies knowledge for performing a specific operation to the neural network.

The neural network may be trained in a direction to minimize errors of an output. The training of the neural network is a process of repeatedly inputting training data into the neural network and calculating the output of the neural network for the training data and the error of a target and back-propagating the errors of the neural network from the output layer of the neural network toward the input layer in a direction to reduce the errors to update the weight of each node of the neural network. In the case of the supervised learning, the training data labeled with a correct answer is used for each training data (i.e., the labeled training data) and in the case of the unsupervised learning, the correct answer may not be labeled in each training data. That is, for example, the training data in the case of the supervised learning related to the data classification may be data in which category is labeled in each training data. The labeled training data is input to the neural network, and the error may be calculated by comparing the output (category) of the neural network with the label of the training data. As another example, in the case of the unsupervised learning related to the data classification, the training data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation. A variation amount of the updated connection weight of each node may be determined according to a learning rate. Calculation of the neural network for the input data and the back-propagation of the error may constitute a training cycle (epoch). The learning rate may be applied differently according to the number of repetition times of the training cycle of the neural network. For example, in an initial stage of the training of the neural network, the neural network ensures a certain level of performance quickly by using a high learning rate, thereby increasing efficiency and uses a low learning rate in a latter stage of the training, thereby increasing accuracy.

In training of the neural network, the training data may be generally a subset of actual data (i.e., data to be processed using the trained neural network), and as a result, there may be a training cycle in which errors for the training data decrease, but the errors for the actual data increase. Overfitting is a phenomenon in which the errors for the actual data increase due to excessive training of the training data. For example, a phenomenon in which the neural network that trains a cat by showing a yellow cat sees a cat other than the yellow cat and does not recognize the corresponding cat as the cat may be a kind of overfitting. The overfitting may act as a cause which increases the error of the machine learning algorithm. Various optimization methods may be used in order to prevent the overfitting. In order to prevent the overfitting, a method such as increasing the training data, regularization, dropout of omitting a part of the node of the network in the process of training, utilization of a batch normalization layer, etc., may be applied.

The present disclosure proposes a model training method using a past biological signal and information associated with the past biological signal in order to improve performance of a model that generates disease prediction information using a neural network model.

The present disclosure has an effect of improving performance of a model that generates disease prediction information for a time point of diagnosis by maximally utilizing a past biological signal measured by an individual patient at a past time point prior to the time point of diagnosis and medical data associated with the past biological signal. In addition, the present disclosure has an effect of improving performance compared with a model that uses only a single biological signal at the time point of diagnosis even when medical data associated with the past biological signal cannot be obtained.

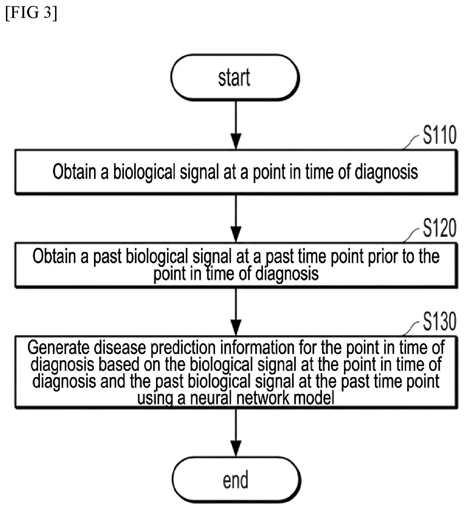

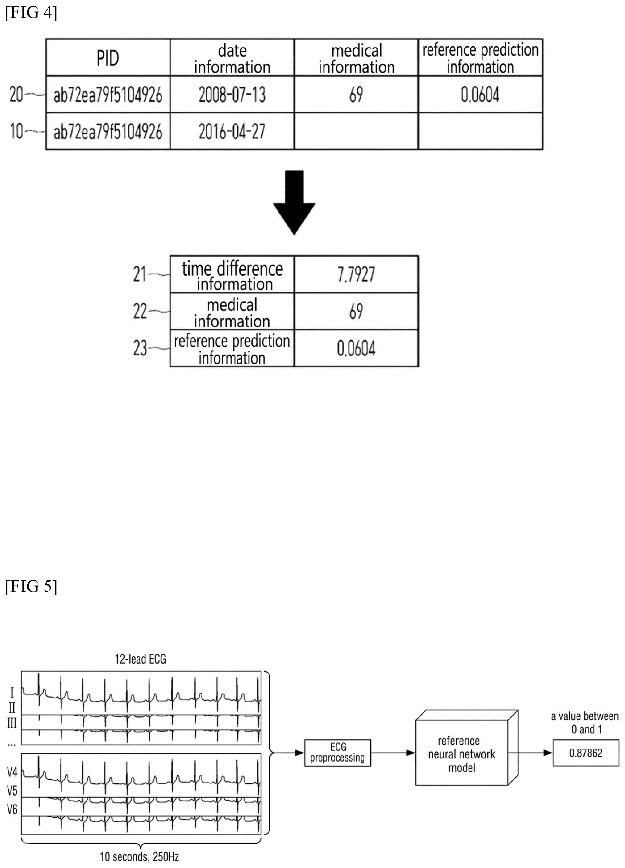

FIG. 3 is a flowchart illustrating a method for generating disease prediction information using a biological signal according to an embodiment of the present disclosure, FIG. 4 is a diagram for explaining an embodiment of information associated with a single past biological signal according to an embodiment of the present disclosure, and FIG. 5 is a diagram for explaining an operation of generating reference prediction information for a past biological signal using a reference neural network model different from the neural network model according to an embodiment of the present disclosure.

A biological signal (Bio-signal) mentioned below may include various biological-related signals such as an electrocardiogram (ECG: electrocardiogram), an electromyogram (EMG: Electromyogram), an electroencephalogram (EEG: electroencephalogram), electrodermal activity (EDA: Electrodermal activity), skin temperature (SKT: skin temperature), photoplethysmography (PPG: photoplethysmography), a vital sign, an X-ray image signal, a magnetic resonance imaging (MRI) signal, and a computed tomography (CT) image signal. In addition, the biological signal is not limited to these examples and may include various types of information acquired from a subject using a medical device, a wearable device, a mobile device, or the like.

Meanwhile, hereinafter, embodiments of the present disclosure will be described mainly with reference to an embodiment in which the biological signal (Bio-signal) is an electrocardiogram (ECG) signal. For example, the present disclosure will be mainly described with reference to an embodiment in which disease prediction information for the time point of diagnosis is generated based on a biological signal at the time point of diagnosis (an ECG signal at the time point of diagnosis) and a past biological signal at a past time point (a past ECG signal) using a neural network model. However, the above-described matters are merely examples and the present disclosure is not limited thereto.

First, with reference to FIGS. 3 to 5, an operation of generating disease prediction information for a time point of diagnosis by obtaining a "past biological signal at a single past time point" in the computing device 100 will be described.

For reference, a "past time point" described below refers to a time point that occurred before the time point of diagnosis, and a unit for the past time point may be divided into, for example, a second, a minute, an hour, a day, a week, a month, or a year.

In addition, the time point of diagnosis and the past time point may include macroscopic or microscopic time information related to measurement of a biological signal. For example, when the unit of the time point is a second, a minute, or an hour, the time point of diagnosis and the past time point may include microscopic time information indicating a start point, a middle point, or an end point of measurement of the biological signal. In addition, when the unit of the time point is a day, a week, a month, or a year, the time point of diagnosis and the past time point may include macroscopic time information including a measurement duration of the biological signal.

Referring to FIG. 3 according to an embodiment of the present disclosure, a method for generating disease prediction information using a biological signal may include obtaining a biological signal at a time point of diagnosis S110, obtaining a past biological signal at a past time point prior to the time point of diagnosis S120, and generating disease prediction information for the time point of diagnosis based on the biological signal at the time point of diagnosis and the past biological signal at the past time point using a neural network model S130. The method for generating disease prediction information using a biological signal illustrated in FIG. 3 may be performed by the computing device 100.

According to an embodiment of the present disclosure, the computing device 100 may obtain a biological signal at a time point of diagnosis (current time point) S110. For example, the computing device 100 may obtain the biological signal at the time point of diagnosis through medical equipment installed in a medical institution or through a portable measurement device (for example, a smart watch, a patch, a bar-type electrocardiogram measurement device, or the like). Alternatively, the computing device 100 may obtain the biological signal at the time point of diagnosis from an electronic medical record (EMR) or the like.

In addition, the computing device 100 may obtain information 10 associated with the biological signal at the time point of diagnosis. For example, referring to FIG. 4, the computing device 100 may obtain, in addition to the biological signal at the time point of diagnosis, unique ID (PID) information associated with the biological signal at the time point of diagnosis, date information for the time point of diagnosis, and the like. In addition, "PID" illustrated in FIG. 4 may be information associated with identification information unique to a patient. However, the information 10 associated with the biological signal at the time point of diagnosis is not limited thereto, and various types of information associated with biological signals may be included.

According to an embodiment of the present disclosure, the computing device 100 may obtain a past biological signal at a past time point prior to the time point of diagnosis S120. In one embodiment, the computing device 100 may obtain the past biological signal at the past time point prior to the time point of diagnosis through a database of a medical institution, a storage of a portable measurement device, or the like. Alternatively, the computing device 100 may obtain the past biological signal at the past time point prior to the time point of diagnosis from an electronic medical record (EMR) or the like.

In addition, the computing device 100 may obtain information 20 associated with the past biological signal at the past time point. For example, referring to FIG. 4, the computing device 100 may obtain, in addition to the past biological signal at the past time point, PID information associated with the past biological signal at the past time point, date information for the past time point, and the like. In addition, "PID" illustrated in FIG. 4 may be information associated with identification information unique to a patient. However, the information 20 associated with the past biological signal at the past time point is not limited thereto, and various types of information associated with biological signals may be included.

In one embodiment, the information 20 associated with the past biological signal may further include at least one of time difference information 21 between the past time point and the time point of diagnosis, medical information 22 mapped to the past biological signal, or reference prediction information 23 generated for the past biological signal using a reference neural network model different from the neural network model.

For example, the time difference information 21 may be information obtained by calculating a time difference between the past time point and the time point of diagnosis. For example, referring to FIG. 4, the computing device 100 may obtain the time difference information 21 based on a difference between a first time included in the information 20 associated with the past biological signal and a second time included in the information 10 associated with the biological signal at the time point of diagnosis. As an example, when the first time is Jul. 13, 2008 and the second time is Apr. 27, 2016, the computing device 100 may obtain time difference information of 7.7927 from the time difference between the second time and the first time. For reference, a unit of the time difference information may be a year.

In addition, the computing device 100 may obtain the medical information 22 mapped to the past biological signal from an electronic medical record (EMR). However, the present disclosure is not limited thereto, and the computing device 100 may obtain medical information mapped to the past biological signal through various methods. For example, the medical information 22 may include medical measurement information measured within a predetermined time from an obtained time of the past biological signal. For example, when the neural network model generating disease prediction information for the time point of diagnosis is a model for diagnosing heart failure, the past biological signal may be an electrocardiogram (ECG) signal, and the medical measurement information may include a left ventricular ejection fraction (EF) value measured within 15 days from a measurement time of the past electrocardiogram signal or information indicating whether heart failure has been diagnosed. In addition, when the neural network model generating disease prediction information for the time point of diagnosis is a model for diagnosing abnormalities in estimated glomerular filtration rate (eGFR) and blood potassium concentration, the past biological signal may be an electrocardiogram signal, and the medical measurement information may include a blood test value measured within one hour from a measurement time of the electrocardiogram signal. However, the present disclosure is not limited thereto and various embodiments may exist.

Meanwhile, the computing device 100 may improve performance of the neural network model that diagnoses the biological signal at the time point of diagnosis by additionally obtaining medical information mapped to the past biological signal. For example, the computing device 100 may implement a neural network model that improves accuracy of disease prediction information by comprehensively considering additional medical information associated with the past biological signal in a prediction process.

According to an embodiment of the present disclosure, the computing device 100 may generate reference prediction information 23 for the past biological signal using a reference neural network model different from the neural network model. For reference, the neural network model is a model that generates disease prediction information for the time point of diagnosis based on the biological signal at the time point of diagnosis and the past biological signal at the past time point (that is, a model that generates disease prediction information for the time point of diagnosis using biological signals of a plurality of time points including the time point of diagnosis). In addition, the reference neural network model may be a separate model configured to generate a reference prediction value based on a biological signal of a single time point. For example, referring to FIG. 5, the computing device 100 may obtain a biological signal of a single time point (for example, an electrocardiogram signal). In addition, the computing device 100 may perform preprocessing on the biological signal of the single time point (for example, an electrocardiogram signal). Thereafter, the computing device 100 may diagnose a specific disease by inputting the preprocessed biological signal of the single time point into the reference neural network model. According to one embodiment, the reference neural network model may be a model trained to diagnose a case in which a left ventricular ejection fraction (ejection fraction, EF) is 40% or less from a biological signal of a single time point obtained using a 12-lead. In addition, the reference neural network model may output a prediction result of a specific disease as a value between 0 and 1. According to such an embodiment of the present disclosure, separate prediction information based on a single time point generated for the past biological signal itself using "a separate neural network model based on a single time point (that is, a separate neural network model that performs diagnosis by analyzing a biological signal of a single time point)" may be additionally included in the information 20 associated with the past biological signal and considered by the neural network model, so that more complex information related to the past biological signal may affect prediction of the neural network model. Accordingly, accuracy of prediction of the neural network model may be further improved through such operations.

According to an embodiment of the present disclosure, the computing device 100 may generate disease prediction information for the time point of diagnosis based on the biological signal at the time point of diagnosis and the past biological signal at the past time point using the neural network model S130.

Here, the neural network model may perform ① an operation based on the biological signal at the time point of diagnosis, an operation based on information 10 associated with the biological signal at the time point of diagnosis, or an operation based on the biological signal at the time point of diagnosis and the information 10 associated with the biological signal at the time point of diagnosis (three optional operations), and may perform, in parallel, ② an operation based on the past biological signal, an operation based on information 20 associated with the past biological signal, or an operation based on the past biological signal and the information 20 associated with the past biological signal (three optional operations), and may finally generate disease prediction information for the time point of diagnosis based on combinations of nine optional operations. Meanwhile, the biological signal at the time point of diagnosis, the information 10 associated with the biological signal at the time point of diagnosis, the past biological signal, or the information 20 associated with the past biological signal may be embedded and vectorized after being input into the neural network model, or may be input into the neural network model in a vectorized state through preprocessing.

In one embodiment, the computing device 100 may i) generate a first vector based on the biological signal at the time point of diagnosis. For example, the computing device 100 may generate the first vector using a feature extraction model. In other words, the computing device 100 may generate the first vector (for example, a feature vector) by inputting the biological signal at the time point of diagnosis into the feature extraction model. For example, the feature extraction model may be implemented as various types of models such as a fully connected layer, a CNN, an RNN, or a transformer.

In addition, the computing device 100 may ii) generate a second vector based on information associated with the past biological signal. For example, referring to FIG. 4, the computing device 100 may generate the second vector based on the information 20 associated with the past biological signal. In one embodiment, the computing device 100 may generate tabular data based on the information 20 associated with the past biological signal and may generate the second vector based on the tabular data. For example, the tabular data may include, as illustrated in FIG. 4, first-type data 21 related to time difference, second-type data 22 related to medical information, and third-type data 23 related to disease prediction based on a single time point.

In addition, the computing device 100 may iii) generate the disease prediction information for the time point of diagnosis based on the first vector and the second vector using the neural network model. For example, the computing device 100 may generate an integrated vector based on the first vector and the second vector. In addition, the computing device 100 may generate the disease prediction information for the time point of diagnosis based on the integrated vector using the neural network model. Here, the first vector and the second vector may include different numbers of elements, and the integrated vector may include more elements of the first vector than elements of the second vector. Since prediction information of the neural network model is ultimately for the time point of diagnosis and information regarding the past time point is merely auxiliary information for improving prediction accuracy for the time point of diagnosis, it is preferable that the integrated vector include more elements of the first vector than elements of the second vector.

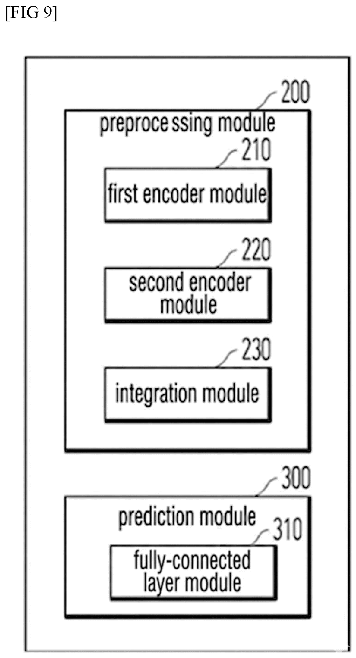

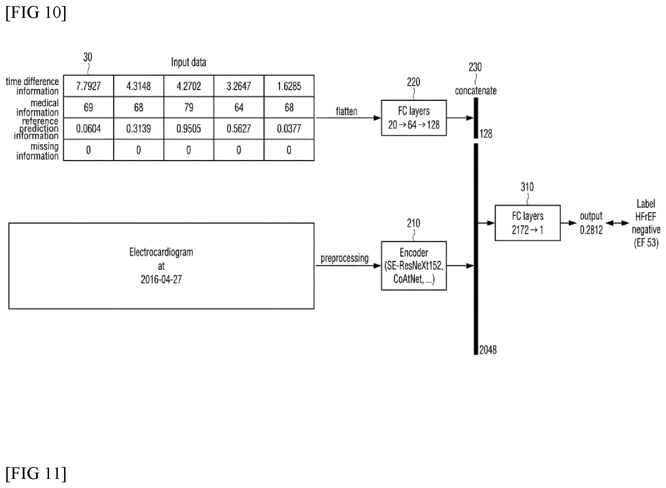

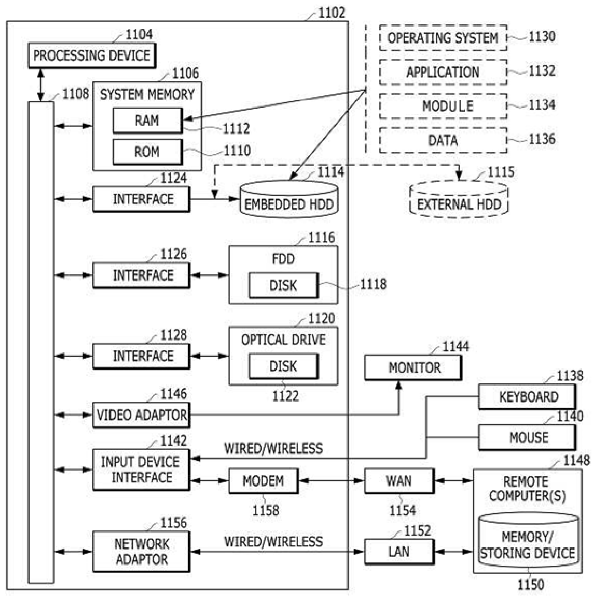

Meanwhile, an operation of generating the disease prediction information for the time point of diagnosis based on the first vector and the second vector using the neural network model will be described in more detail with reference to FIGS. 9 and 10 below.

Next, with reference to FIG. 3 and FIGS. 6 to 8, an operation of generating disease prediction information for a time point of diagnosis by obtaining "a plurality of past biological signals" in the computing device 100 will be described.

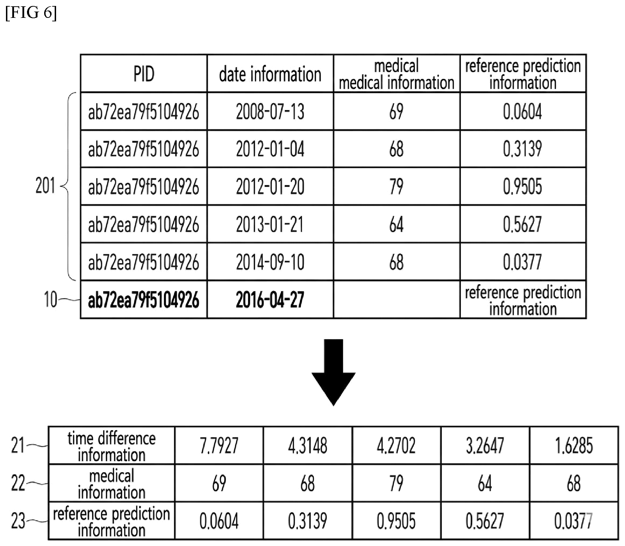

FIG. 6 is a diagram for explaining information associated with a plurality of past biological signals according to an embodiment of the present disclosure.

Referring again to FIG. 3 according to an embodiment of the present disclosure, a method for generating disease prediction information using a biological signal may include obtaining a biological signal at a time point of diagnosis S110, obtaining a past biological signal at a past time point prior to the time point of diagnosis S 120, and generating disease prediction information for the time point of diagnosis based on the biological signal at the time point of diagnosis and the past biological signal at the past time point using a neural network model S130. The method for generating disease prediction information using a biological signal illustrated in FIG. 3 may be performed by the computing device 100.

Meanwhile, in embodiments to be described below, with respect to step S120, a plurality of past biological signals of a plurality of past time points (rather than a single past biological signal of a single time point) may be obtained. In addition, in step S130, the plurality of past biological signals may be considered in a process of generating the disease prediction information.

According to an embodiment of the present disclosure, the computing device 100 may obtain a biological signal at a time point of diagnosis S110. For example, the computing device 100 may obtain the biological signal at the time point of diagnosis through medical equipment installed in a medical institution or through a portable measurement device (for example, a smart watch, a patch, a bar-type electrocardiogram measurement device, or the like). Alternatively, the computing device 100 may obtain the biological signal at the time point of diagnosis from an electronic medical record EMR or the like.

In addition, the computing device 100 may obtain information 10 associated with the biological signal at the time point of diagnosis. For example, referring to FIG. 6, the computing device 100 may obtain, in addition to the biological signal at the time point of diagnosis, unique ID PID information related to the biological signal at the time point of diagnosis, date information for the time point of diagnosis, and the like. Meanwhile, in a medical record, "PID" may be information associated with identification information unique to a patient. However, the information 10 associated with the biological signal at the time point of diagnosis is not limited thereto, and various types of information associated with biological signals may be included.

According to an embodiment of the present disclosure, the computing device 100 may obtain a past biological signal at a past time point prior to the time point of diagnosis S120. In this case, the computing device 100 may obtain "a plurality of past biological signals for a plurality of past time points" prior to the time point of diagnosis. In one embodiment, the computing device 100 may obtain the plurality of past biological signals of the plurality of past time points prior to the time point of diagnosis through a database of a medical institution, a storage of a portable measurement device, or the like. Alternatively, the computing device 100 may obtain the plurality of past biological signals for the plurality of past time points prior to the time point of diagnosis from an electronic medical record EMR or the like.

In addition, the computing device 100 may obtain information 201 associated with the plurality of past biological signals of the plurality of past time points. For example, referring to FIG. 6, the computing device 100 may obtain, in addition to the plurality of past biological signals of the plurality of past time points, PID information related to each past biological signal, date information for each past time point, and the like. In addition, "PID" illustrated in FIG. 6 may be information associated with identification information unique to a patient. However, the information 201 associated with the plurality of past biological signals is not limited thereto, and various types of information associated with biological signals may be included.

In one embodiment, the information 201 associated with the plurality of past biological signals may include respective additional individual information associated with each past biological signal included in the plurality of past biological signals. The respective additional individual information associated with each past biological signal may include at least one of time difference information 21 between each past time point and the time point of diagnosis, medical information 22 mapped to each past biological signal, or reference prediction information 23 generated for each past biological signal using a reference neural network model different from the neural network model.

Here, the time difference information 21 may be time difference information between each past time point and the time point of diagnosis. For example, referring to FIG. 6, the computing device 100 may obtain the time difference information 21 based on differences between respective times included in the information 201 associated with the plurality of past biological signals and a time included in the information 10 associated with the biological signal at the time point of diagnosis. As an example, when a first time included in the information associated with the biological signal at the time point of diagnosis is Apr. 27, 2016 and a second time included in the information 201 associated with the plurality of past biological signals is Jul. 13, 2008, the computing device 100 may obtain time difference information of 7.7927 from a time difference between the second time and the first time. In the same manner, when a third time included in the information 201 associated with the plurality of past biological signals is Jan. 14, 2012, the computing device 100 may obtain time difference information of 4.3148 from a time difference between the third time and the first time. The computing device 100 may generate a plurality of time difference information items between the plurality of past time points included in the information 201 associated with the plurality of past biological signals and the time point of diagnosis.

In addition, the computing device 100 may obtain each medical information 22 mapped to each past biological signal from an electronic medical record (electronic medical record; EMR). However, the present disclosure is not limited thereto, and the computing device 100 may obtain each medical information mapped to each past biological signal through various methods. For example, the medical information 22 may include each medical measurement information measured within a predetermined time from an obtained time of each past biological signal. For example, when the neural network model generating disease prediction information for the time point of diagnosis is a model for diagnosing heart failure, each past biological signal may be an electrocardiogram ECG signal, and each medical measurement information may include a left ventricular ejection fraction EF value measured within 15 days from a measurement time of the electrocardiogram ECG signal obtained in the past or information indicating whether heart failure has been diagnosed. In addition, when the neural network model generating disease prediction information for the time point of diagnosis is a model for diagnosing abnormalities in estimated glomerular filtration rate eGFR and blood potassium concentration, each past biological signal may be an electrocardiogram ECG signal, and each medical measurement information may include a blood test value measured within one hour from a measurement time of the electrocardiogram ECG signal. However, the present disclosure is not limited thereto, and various embodiments may exist. Meanwhile, the computing device 100 may improve performance of the neural network model that diagnoses the biological signal at the time point of diagnosis by additionally obtaining each medical information mapped to each past biological signal. For example, the computing device 100 may implement a neural network model that improves accuracy of disease prediction information by comprehensively considering additional medical information related to the past biological signal in a prediction process.

According to one embodiment, the computing device 100 may generate each reference prediction information 23 for each past biological signal using a reference neural network model different from the neural network model. For reference, the neural network model is a model that generates disease prediction information for the time point of diagnosis based on the biological signal at the time point of diagnosis and the plurality of past biological signals of the plurality of past time points. In addition, the reference neural network model may be a separate model configured to generate a reference prediction value based on a biological signal of a single time point. For example, referring again to FIG. 5, the computing device 100 may obtain a biological signal of a single time point (for example, an electrocardiogram signal). In addition, the computing device 100 may perform preprocessing on the biological signal of the single time point (for example, an electrocardiogram signal). Thereafter, the computing device 100 may diagnose a specific disease by inputting the preprocessed biological signal of the single time point into the reference neural network model. According to one embodiment, the reference neural network model may be a model trained to diagnose a case in which a left ventricular ejection fraction (ejection fraction, EF) is 40% or less from a biological signal of a single time point obtained using a 12-lead. The reference neural network model may output a prediction result of a specific disease as a value between 0 and 1. As described above, according to such an embodiment of the present disclosure, a plurality of separate prediction information items based on a single time point generated for each past biological signal itself using "a separate neural network model based on a single time point" may be additionally included in the information 201 associated with the plurality of past biological signals and may be considered by the neural network model, so that more complex information related to the plurality of past biological signals may affect prediction of the neural network model. Accordingly, accuracy of prediction of the neural network model may be further improved through such operations.

According to an embodiment of the present disclosure, the computing device 100 may generate disease prediction information for the time point of diagnosis based on the biological signal at the time point of diagnosis and the past biological signal at the past time point using the neural network model S130. In particular, the computing device 100 may generate disease prediction information for the time point of diagnosis based on the biological signal at the time point of diagnosis and the plurality of past biological signals of the plurality of past time points using the neural network model.

Here, the neural network model may perform ① an operation based on the biological signal at the time point of diagnosis, an operation based on information 10 associated with the biological signal at the time point of diagnosis, or an operation based on the biological signal at the time point of diagnosis and the information 10 associated with the biological signal at the time point of diagnosis (three optional operations), and may perform, in parallel, ② an operation based on the plurality of past biological signals, an operation based on information 201 associated with the plurality of past biological signals, or an operation based on the plurality of past biological signals and information 201 associated with the plurality of past biological signals (three optional operations), and may finally generate disease prediction information for the time point of diagnosis based on combinations of nine optional operations. Meanwhile, the biological signal at the time point of diagnosis, the information 10 associated with the biological signal at the time point of diagnosis, the plurality of past biological signals, or the information 201 associated with the plurality of past biological signals may be embedded and vectorized after being input into the neural network model, or may be input into the neural network model in a vectorized state through preprocessing.

In one embodiment, the computing device 100 may i) generate a first vector based on the biological signal at the time point of diagnosis. For example, the computing device 100 may generate the first vector using a feature extraction model. In other words, the computing device 100 may generate the first vector (for example, a feature vector) by inputting the biological signal at the time point of diagnosis into the feature extraction model. For example, the feature extraction model may be implemented as various types of models such as a fully connected layer, a CNN, an RNN, or a transformer.

In addition, the computing device 100 may ii) generate a second vector based on information associated with the plurality of past biological signals. For example, referring to FIG. 6, the computing device 100 may generate the second vector based on the information 201 associated with the plurality of past biological signals. In one embodiment, the computing device 100 may generate tabular data based on the information 201 associated with the plurality of past biological signals and may generate the second vector based on the tabular data. For example, the tabular data may include, as illustrated in FIG. 6, first-type data 21 related to time difference, second-type data 22 related to medical information, and third-type data 23 related to disease prediction based on a single time point.

According to an embodiment of the present disclosure, the neural network model may include a network that performs disease prediction using information associated with a biological signal at a time point of diagnosis and information associated with a predetermined number (for example, five) of past biological signals.

In this case, in order for the neural network model to perform a normal prediction operation, the predetermined number of past biological signals needs to be secured, but in some situations (for example, a situation in which a past measurement history is insufficient, a situation in which a new patient performs measurement, a situation in which a loss occurs in a database, or the like), it may be difficult to secure the predetermined number of past biological signals. Therefore, even in such a missing situation of past biological signals, an operation of processing missing information capable of ensuring normal operation of the neural network model is required.

In one embodiment, when it is difficult to secure the predetermined number of past biological signals, the computing device 100 may generate supplementary biological signals for complementing a deficient number of missing signals. For example, the computing device 100 may generate the deficient number of supplementary biological signals using at least one obtained past biological signal. Specifically, the computing device 100 may generate the deficient number of supplementary biological signals by copying at least one obtained past biological signal and information associated therewith as they are, or may generate the deficient number of supplementary biological signals by combining two or more obtained past biological signals, or may generate the deficient number of supplementary biological signals through various methods other than these methods.

Meanwhile, the computing device 100 may generate the deficient number of supplementary biological signals based on a past biological signal of the earliest past time point among the obtained past biological signals. For example, the computing device 100 may generate the deficient number of supplementary biological signals by copying the past biological signal of the earliest past time point and information associated therewith. When the complementing operation is performed in this manner, computation may be simplified so that computational resources may be efficiently managed, and it is possible to prevent the neural network model from performing prediction while excessively concentrating on information having a low temporal distribution near the time point of diagnosis.

In addition, the computing device 100 may add data related to missing information to the information 201 associated with the plurality of biological signals. For example, after generating the deficient number of supplementary biological signals, the computing device 100 may add, to the information 201 associated with the plurality of biological signals, data indicating that each supplementary biological signal is complementarily generated according to a missing situation. In one embodiment, the information 201 associated with the plurality of biological signals may be implemented as the tabular data described above, and in this case, the tabular data may further include fourth-type data related to missing information. Here, the fourth-type data may indicate whether a corresponding past biological signal is an actually obtained past biological signal or a supplementary biological signal.

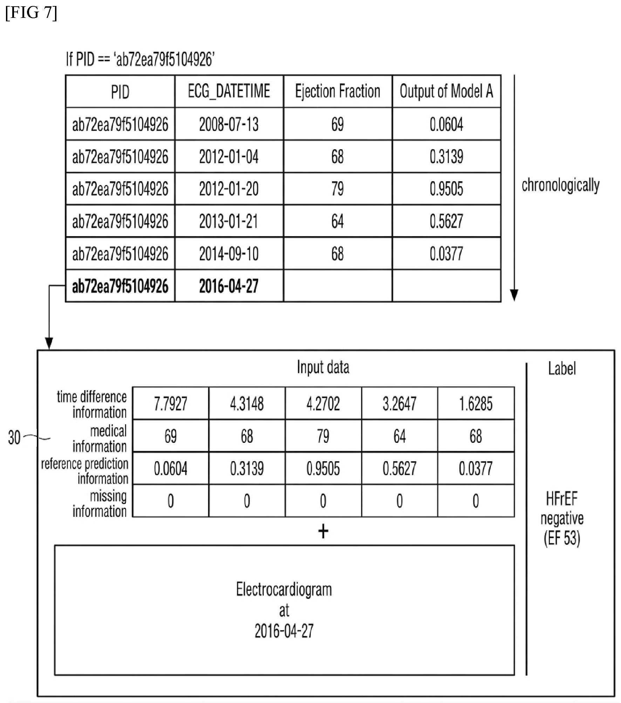

FIG. 7 is a diagram for explaining an operation of generating a second vector when the number of a plurality of past biological signals satisfies a predetermined number according to an embodiment of the present disclosure. In other words, the embodiment of FIG. 7 is an example showing a situation in which the neural network model operates depending on a predetermined number of past biological signals and all of the predetermined number of past biological signals are obtained (that is, a situation in which there is no missing value).

Referring to FIG. 7, the computing device 100 may obtain a predetermined number of past biological signals (for example, five) and may generate tabular data 30 based on the obtained past biological signals. Here, the tabular data 30 may include information associated with the predetermined number of past biological signals. In addition, the computing device 100 may generate the second vector for the predetermined number of past biological signals based on the tabular data 30.

In addition, the computing device 100 may generate an integrated vector based on the first vector and the second vector using the neural network model, and may generate the disease prediction information for the time point of diagnosis based on the integrated vector. Here, the first vector and the second vector may include different numbers of elements, and the integrated vector may include more elements of the first vector than elements of the second vector. That is, the neural network model may generate the integrated vector by placing more weight on the biological signal at the time point of diagnosis than on a plurality of past biological signals at a plurality of past time points, and may predict a disease using such an integrated vector.

As an example, referring to FIG. 7, the first vector may be generated based on the biological signal at the time point of diagnosis corresponding to Apr. 27, 2016. In addition, the second vector may be generated based on information associated with each of a predetermined number of past biological signals obtained at a predetermined number of past time points (for example, Sep. 10, 2014, Jan. 21, 2013, Jan. 20, 2012, Jan. 04, 2012, and Jul. 13, 2008) prior to Apr. 27, 2016 (the time point of diagnosis). For example, the computing device 100 may generate tabular data 30 based on the predetermined number of past biological signals and may generate the second vector based on the tabular data 30. Meanwhile, an operation of generating the disease prediction information for the time point of diagnosis based on the first vector and the second vector using the neural network model will be described in more detail with reference to FIGS. 9 and 10 below.

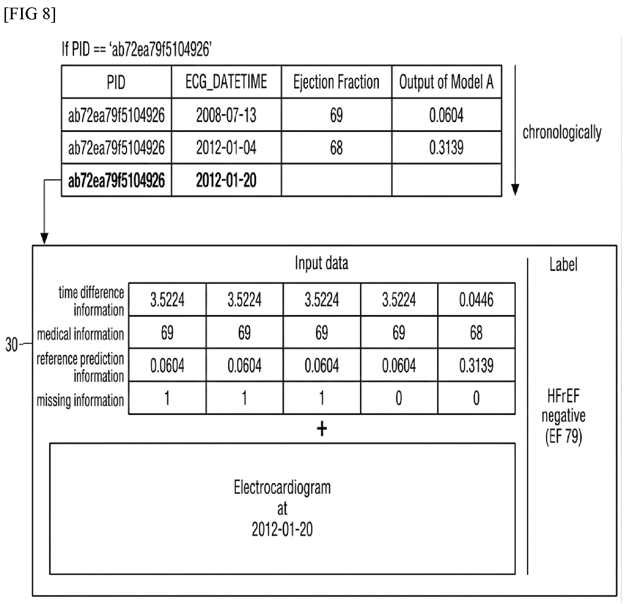

FIG. 8 is a diagram for explaining an operation of generating a second vector when the number of a plurality of past biological signals is less than a predetermined number according to an embodiment of the present disclosure. In other words, the embodiment of FIG. 8 is an example showing a situation in which the neural network model operates depending on a predetermined number of past biological signals and the number of obtained past biological signals is less than the predetermined number (that is, a situation in which there is a missing value).

Referring to FIG. 8, when "the number of obtained past biological signals is less than a predetermined number (for example, five)", the computing device 100 may generate a deficient number of supplementary biological signals using at least one past biological signal among the obtained past biological signals. For example, the supplementary biological signals may be generated based on a past biological signal of the earliest past time point among the obtained past biological signals. In addition, the computing device 100 may generate the second vector based on information associated with the obtained past biological signals and information associated with the supplementary biological signals. For example, the computing device 100 may generate the second vector based on tabular data 30 as illustrated in FIG. 8, the tabular data 30 including information associated with the obtained past biological signals and information associated with the supplementary biological signals.

In addition, the computing device 100 may generate an integrated vector based on the first vector and the second vector using the neural network model, and may generate the disease prediction information for the time point of diagnosis based on the integrated vector. Here, the first vector and the second vector may include different numbers of elements, and the integrated vector may include more elements of the first vector than elements of the second vector. That is, the neural network model may generate the integrated vector by placing more weight on the biological signal at the time point of diagnosis than on a plurality of past biological signals at a plurality of past time points, and may predict a disease using such an integrated vector.

As an example, referring to FIG. 8, the first vector may be generated based on the biological signal at the time point of diagnosis corresponding to Apr. 27, 2016. In addition, when the predetermined number is five, the second vector may be generated based on information associated with two past biological signals obtained at two past time points (Jan. 04, 2012 and Jul. 13, 2008) prior to Apr. 27, 2016 (the time point of diagnosis), and information associated with three supplementary biological signals generated for complementing. In addition, the information associated with the three supplementary biological signals may all be generated based on a past biological signal obtained on Jul. 13, 2008, which is the earliest past time point among the two obtained past biological signals. In one embodiment, the information associated with the three supplementary biological signals may be generated by copying first-type data (for example, time difference information), second-type data (for example, medical information), and third-type data of the past biological signal obtained on Jul. 13, 2008, which is the earliest past time point, as they are, and setting fourth-type data to an opposite value. Meanwhile, an operation of generating the disease prediction information for the time point of diagnosis based on the first vector and the second vector using the neural network model will be described in more detail with reference to FIGS. 9 and 10 below.

Hereinafter, an operation of generating the disease prediction information for the time point of diagnosis based on the first vector and the second vector using the neural network model will be described in more detail with reference to FIGS. 9 and 10.

FIG. 9 is a block diagram of a structure of a neural network model for generating disease prediction information using a biological signal according to an embodiment of the present disclosure, and FIG. 10 is a diagram schematically illustrating an operation of generating disease prediction information in the neural network model according to an embodiment of the present disclosure. For reference, in FIG. 10, the obtained biological signal at the time point of diagnosis and the past biological signal at a past time point prior to the time point of diagnosis may be electrocardiogram ECG signals. In addition, the neural network model may be a model for diagnosing a case in which a left ventricular ejection fraction (ejection fraction, EF) is 40% or less using an electrocardiogram signal. However, the present disclosure is not limited thereto, and the neural network model may be variously implemented as a model for generating disease prediction information for a specific disease corresponding to various biological signals to be obtained.

Referring to FIG. 9, the structure of the neural network model may include a preprocessing module 200 and a prediction module 300.

According to an embodiment of the present disclosure, the preprocessing module 200 may preprocess a biological signal at a time point of diagnosis and a past biological signal at a past time point prior to the time point of diagnosis. For example, the preprocessing module 200 may include a first encoder module 210, a second encoder module 220, and an integration module 230.

According to one embodiment, the first encoder module 210 may generate a first vector based on the biological signal at the time point of diagnosis. For example, referring to FIG. 10, the biological signal at the time point of diagnosis may be an electrocardiogram ECG signal measured on Apr. 27, 2016. In one embodiment, after receiving the preprocessed electrocardiogram signal at the time point of diagnosis, the first encoder module 210 may generate the first vector using various neural network architectures such as SE-ResNeXt 152 and CoAtNet. Alternatively, the first encoder module 210 may generate the first vector using various neural network architectures such as a fully connected layer, a CNN, an RNN, and a transformer. Meanwhile, in the example of FIG. 10, the first vector may be implemented in a form of a vector including 2048 elements, but the number of elements of the first vector is not limited to this example and may be variously configured under a condition that the first vector includes more elements than elements of the second vector.

In addition, the second encoder module 220 may generate a second vector based on the past biological signal. For example, the second encoder module 220 may generate the second vector based on information associated with a plurality of past biological signals. For example, referring to FIG. 10, the second encoder module 220 may obtain tabular data 30 generated based on the plurality of past biological signals. Here, the tabular data 30 may include first-type data related to time difference, second-type data related to medical information, third-type data related to disease prediction based on a single time point, fourth-type data related to missing information, and the like. For example, as described above, the tabular data 30 may be generated i) based on obtained past biological signals when "the number of obtained past biological signals satisfies a predetermined number (for example, five)", or ii) may be generated based on information associated with the obtained past biological signals and information associated with supplementary biological signals by generating a deficient number of supplementary biological signals using at least one past biological signal among the obtained past biological signals when "the number of obtained past biological signals is less than a predetermined number (for example, five)".

In addition, the second encoder module 220 may perform a flatten operation of converting the tabular data 30 into one dimension. For example, the second encoder module 220 may convert the tabular data 30 into a one-dimensional vector through a flatten operation, and may generate the second vector after additional processing such as resizing. In one embodiment, the second encoder module 220 may include a second-1 encoder layer, a second-2 encoder layer, and a second-3 encoder layer sequentially connected. In addition, the second-1 encoder layer may include 20 neurons, the second-2 encoder layer may include 64 neurons, and the second-3 encoder layer may include 128 neurons, and the second vector including 128 elements may be generated using these sequential layers. Meanwhile, the second encoder module 220 may generate the second vector using various neural network architectures such as a fully connected layer, a CNN, an RNN, and a transformer.

In addition, the integration module 230 may generate an integrated vector based on the first vector and the second vector. For example, the integration module 230 may generate the integrated vector based on a summation of the first vector and the second vector, may generate the integrated vector based on a weighted sum of the first vector and the second vector, may generate the integrated vector by concatenating the first vector and the second vector, may generate the integrated vector by performing element-wise multiplication on the first vector and the second vector, may generate the integrated vector by performing average pooling or max pooling on the first vector and the second vector, or may generate the integrated vector by applying an attention mechanism between the first vector and the second vector, and may generate the integrated vector using various methods other than these methods.

Here, the first vector and the second vector may include different numbers of elements, and the integrated vector may include more elements of the first vector than elements of the second vector. That is, the integrated vector may be generated in a form placing more weight on the biological signal at the time point of diagnosis than on information associated with a plurality of past biological signals. For example, referring to FIG. 10, the integrated vector may be generated based on 2048 elements of the first vector and 128 elements of the second vector. However, the present disclosure is not limited thereto, and various embodiments may exist.

According to an embodiment of the present disclosure, the prediction module 300 may generate disease prediction information based on data related to the biological signal generated by the preprocessing module 200 and data related to the past biological signal generated by the preprocessing module 200. For example, the prediction module 300 may include a fully connected layer module 310 configured to generate the disease prediction information based on the integrated vector, and may include various alternative neural network architectures in addition to the fully connected layer module 310. Referring to FIG. 10, the fully connected layer module 310 may extract various features of input data using a first FC layer having 2172 neurons and may generate intermediate features having high expressiveness. Such intermediate features may be transferred to a second FC layer and connected to a single output neuron for final prediction. An activation function such as a sigmoid and a softmax used in a classification task may be applied to the second FC layer. However, the present disclosure is not limited thereto. In one embodiment, the prediction module 300 may generate a prediction value between 0 and 1 for diagnosing HFrEF.

Table 1 shows experimental results obtained by evaluating performance of a model implemented according to an embodiment of the present disclosure, namely "a model for diagnosing heart failure (HFrEF) using an electrocardiogram (ECG) signal." In the experiment, labels were set as HFrEF (1) and non-HFrEF (0), and training was performed based on binary classification. Performance was evaluated using a test set separated from the training data. In addition, performance of the model was evaluated based on an AUROC (Area Under the Receiver Operating Characteristic) metric.

Referring to Table 1, it was confirmed that performance improved under all conditions based on the AUROC metric when disease prediction information was generated using the neural network model according to an embodiment of the present disclosure, compared with a conventional model using a single-time-point electrocardiogram (single ECG) signal (that is, a model using only the biological signal at the time point of diagnosis). For example, performance improvement was confirmed in all of the following cases:
① an example in which information associated with five past biological signals is additionally used together with the biological signal at the time point of diagnosis and the associated information includes past medical information EF (ejection fraction) (K=5, past EF used),
② an example in which information associated with five past biological signals is additionally used together with the biological signal at the time point of diagnosis and the associated information does not include past medical information EF (ejection fraction) (K=5, past EF not used),
③ an example in which information associated with three past biological signals is additionally used together with the biological signal at the time point of diagnosis and the associated information includes past medical information EF (ejection fraction) (K=3, past EF used), and
④ an example in which information associated with three past biological signals is additionally used together with the biological signal at the time point of diagnosis and the associated information does not include past medical information EF (ejection fraction) (K=3, past EF not used).

In particular, even under conditions in which past medical information (EF; ejection fraction) history was not used, performance improvement compared to the single-time-point-based model was confirmed in both internal validation and external validation.

**[Table 1]**

| | | Internal validation | External validation |
|---|---|---|---|
| Single ECG model | | 0.9398 | 0.9085 |
| Proposed model (K=5) | Used EF in the past | 0.9556 | 0.9425 |
| | Not using EF in the past | 0.9436 | 0.9192 |
| Proposed model (K=3) | Used EF in the past | 0.9570 | 0.9392 |
| | Not using EF in the past | 0.9496 | 0.9200 |

In the above description, steps S110 to S130 may, according to an embodiment of the present disclosure, be further divided into additional steps or may be combined into fewer steps. In addition, some steps may be omitted as necessary, and an order of the steps may be changed.

Disclosed is a computer readable medium storing the data structure according to an exemplary embodiment of the present disclosure.

The data structure may refer to the organization, management, and storage of data that enables efficient access to and modification of data. The data structure may refer to the organization of data for solving a specific problem (e.g., data search, data storage, data modification in the shortest time). The data structures may be defined as physical or logical relationships between data elements, designed to support specific data processing functions. The logical relationship between data elements may include a connection between data elements that the user defines. The physical relationship between data elements may include an actual relationship between data elements physically stored on a computer-readable storage medium (e.g., persistent storage device). The data structure may specifically include a set of data, a relationship between the data, a function which may be applied to the data, or instructions. Through an availablely designed data structure, a computing device can perform operations while using the resources of the computing device to a minimum. Specifically, the computing device can increase the efficiency of operation, read, insert, delete, compare, exchange, and search through the availablely designed data structure.

The data structure may be divided into a linear data structure and a non-linear data structure according to the type of data structure. The linear data structure may be a structure in which only one data is connected after one data. The linear data structure may include a list, a stack, a queue, and a deque. The list may mean a series of data sets in which an order exists internally. The list may include a linked list. The linked list may be a data structure in which data is connected in a scheme in which each data is linked in a row with a pointer. In the linked list, the pointer may include link information with next or previous data. The linked list may be represented as a single linked list, a double linked list, or a circular linked list depending on the type. The stack may be a data listing structure with limited access to data. The stack may be a linear data structure that may process (e.g., insert or delete) data at only one end of the data structure. The data stored in the stack may be a data structure (LIFO-Last in First Out) in which the data is input last and output first. The queue is a data listing structure that may access data limitedly and unlike a stack, the queue may be a data structure (FIFO-First in First Out) in which late stored data is output late. The deque may be a data structure capable of processing data at both ends of the data structure.

The non-linear data structure may be a structure in which a plurality of data are connected after one data. The non-linear data structure may include a graph data structure. The graph data structure may be defined as a vertex and an edge, and the edge may include a line connecting two different vertices. The graph data structure may include a tree data structure. The tree data structure may be a data structure in which there is one path connecting two different vertices among a plurality of vertices included in the tree. That is, the tree data structure may be a data structure that does not form a loop in the graph data structure.

In the present disclosure, a network function, computation model, and a neural network may be used to be exchangeable. From here on, it will be described uniformly using neural networks.

The data structure may include the neural network. In addition, the data structures, including the neural network, may be stored in a computer readable medium. The data structure including the neural network may also include data preprocessed for processing by the neural network, data input to the neural network, weights of the neural network, hyper parameters of the neural network, data obtained from the neural network, an active function associated with each node or layer of the neural network, and a loss function for training the neural network. The data structure including the neural network may include predetermined components of the components disclosed above. In other words, the data structure including the neural network may include all of data preprocessed for processing by the neural network, data input to the neural network, weights of the neural network, hyper parameters of the neural network, data obtained from the neural network, an active function associated with each node or layer of the neural network, and a loss function for training the neural network or a combination thereof. In addition to the above-described configurations, the data structure including the neural network may include predetermined other information that determines the characteristics of the neural network. In addition, the data structure may include all types of data used or generated in the calculation process of the neural network, and is not limited to the above. The computer readable medium may include a computer readable recording medium and/or a computer readable transmission medium. The neural network may be generally constituted by an aggregate of calculation units which are mutually connected to each other, which may be called nodes. The nodes may also be called neurons. The neural network is configured to include one or more nodes.

The data structure may include data input into the neural network. The data structure including the data input into the neural network may be stored in the computer readable medium. The data input to the neural network may include training data input in a neural network training process and/or input data input to a neural network in which training is completed. The data input to the neural network may include preprocessed data and/or data to be preprocessed. The preprocessing may include a data processing process for inputting data into the neural network. Therefore, the data structure may include data to be preprocessed and data generated by preprocessing. The data structure is just an example and the present disclosure is not limited thereto.

The data structure may include the weight of the neural network (in the present disclosure, the weight and the parameter may be used as the same meaning). In addition, the data structures, including the weight of the neural network, may be stored in the computer readable medium. The neural network may include a plurality of weights. The weight may be variable and the weight is variable by a user or an algorithm in order for the neural network to perform a desired function. For example, when one or more input nodes are mutually connected to one output node by the respective links, the output node may determine a data value output from an output node based on values input in the input nodes connected with the output node and the weights set in the links corresponding to the respective input nodes. The data structure is just an example and the present disclosure is not limited thereto.

As a non-limiting example, the weight may include a weight which varies in the neural network training process and/or a weight in which neural network training is completed. The weight which varies in the neural network training process may include a weight at a time when a training cycle starts and/or a weight that varies during the training cycle. The weight in which the neural network training is completed may include a weight in which the training cycle is completed. Accordingly, the data structure including the weight of the neural network may include a data structure including the weight which varies in the neural network training process and/or the weight in which neural network training is completed. Accordingly, the above-described weight and/or a combination of each weight are included in a data structure including a weight of a neural network. The data structure is just an example and the present disclosure is not limited thereto.

The data structure including the weight of the neural network may be stored in the computer-readable storage medium (e.g., memory, hard disk) after a serialization process. Serialization may be a process of storing data structures on the same or different computing devices and later reconfiguring the data structure and converting the data structure to a form that may be used. The computing device may serialize the data structure to send and receive data over the network. The data structure including the weight of the serialized neural network may be reconfigured in the same computing device or another computing device through deserialization. The data structure including the weight of the neural network is not limited to the serialization. Furthermore, the data structure including the weight of the neural network may include a data structure (for example, B-Tree, Trie, m-way search tree, AVL tree, and Red-Black Tree in a nonlinear data structure) to increase the efficiency of operation while using resources of the computing device to a minimum. The above-described matter is just an example and the present disclosure is not limited thereto.

The data structure may include hyper-parameters of the neural network. In addition, the data structures, including the hyper-parameters of the neural network, may be stored in the computer readable medium. The hyper-parameter may be a variable which may be varied by the user. The hyper-parameter may include, for example, a learning rate, a cost function, the number of training cycle iterations, weight initialization (for example, setting a range of weight values to be subjected to weight initialization), and Hidden Unit number (e.g., the number of hidden layers and the number of nodes in the hidden layer). The data structure is just an example and the present disclosure is not limited thereto.

FIG. 11 is a normal and schematic view of an exemplary computing environment in which the exemplary embodiments of the present disclosure may be implemented.

It is described above that the present disclosure may be generally implemented by the computing device, but those skilled in the art will well know that the present disclosure may be implemented in association with a computer executable command which may be executed on one or more computers and/or in combination with other program modules and/or a combination of hardware and software.

In general, the program module includes a routine, a program, a component, a data structure, and the like that execute a specific task or implement a specific abstract data type. Further, it will be well appreciated by those skilled in the art that the method of the present disclosure can be implemented by other computer system configurations including a personal computer, a handheld computing device, microprocessor-based or programmable home appliances, and others (the respective devices may operate in connection with one or more associated devicesas well as a single-processor or multi-processor computer system, a mini computer, and a main frame computer.

The exemplary embodiments described in the present disclosure may also be implemented in a distributed computing environment in which predetermined tasks are performed by remote processing devices connected through a communication network. In the distributed computing environment, the program module may be positioned in both local and remote memory storage devices.
The computer generally includes various computer readable media. Media accessible by the computer may be computer readable media regardless of types thereof and the computer readable media include volatile and non-volatile media, transitory and non-transitory media, and mobile and non-mobile media. As a non-limiting example, the computer readable media may include both computer readable storage media and computer readable transmission media. The computer readable storage media include volatile and non-volatile media, transitory and non-transitory media, and mobile and non-mobile media implemented by a predetermined method or technology for storing information such as a computer readable instruction, a data structure, a program module, or other data. The computer readable storage media include a RAM, a ROM, an EEPROM, a flash memory or other memory technologies, a CD-ROM, a digital video disk (DVD) or other optical disk storage devices, a magnetic cassette, a magnetic tape, a magnetic disk storage device or other magnetic storage devices or predetermined other media which may be accessed by the computer or may be used to store desired information, but are not limited thereto.
The computer readable transmission media generally implement the computer readable command, the data structure, the program module, or other data in a carrier wave or a modulated data signal such as other transport mechanism and include all information transfer media. The term "modulated data signal" means a signal acquired by setting or changing at least one of characteristics of the signal so as to encode information in the signal. As a non-limiting example, the computer readable transmission media include wired media such as a wired network or a direct-wired connection and wireless media such as acoustic, RF, infrared and other wireless media. A combination of anymedia among the aforementioned media is also included in a range of the computer readable transmission media.

An exemplary environment 1100 that implements various aspects of the present disclosure including a computer 1102 is shown and the computer 1102 includes a processing device 1104, a system memory 1106, and a system bus 1108. The system bus 1108 connects system components including the system memory 1106 (not limited thereto) to the processing device 1104. The processing device 1104 may be a predetermined processor among various commercial processors. A dual processor and other multi-processor architectures may also be used as the processing device 1104.

The system bus 1108 may be any one of several types of bus structures which may be additionally interconnected to a local bus using any one of a memory bus, a peripheral device bus, and various commercial bus architectures. The system memory 1106 includes a read only memory (ROM) 1110 and a random access memory (RAM) 1112. A basic input/output system (BIOS) is stored in the non-volatile memories 1110 including the ROM, the EPROM, the EEPROM, and the like and the BIOS includes a basic routine that assists in transmitting information among components in the computer 1102 at a time such as in-starting. The RAM 1112 may also include a high-speed RAM including a static RAM for caching data, and the like.

The computer 1102 also includes an interior hard disk drive (HDD) 1114 (for example, EIDE and SATA), in which the interior hard disk drive 1114 may also be configured for an exterior purpose in an appropriate chassis (not illustrated), a magnetic floppy disk drive (FDD) 1116 (for example, for reading from or writing in a mobile diskette 1118), and an optical disk drive 1120 (for example, for reading a CD-ROM disk 1122 or reading from or writing in other high-capacity optical media such as the DVD, and the like). The hard disk drive 1114, the magnetic disk drive 1116, and the optical disk drive 1120 may be connected to the system bus 1108 by a hard disk drive interface 1124, a magnetic disk drive interface 1126, and an optical drive interface 1128, respectively. An interface 1124 for implementing an exterior drive includes at least one of a universal serial bus (USB) and an IEEE 1394 interface technology or both of them. The drives and the computer readable media associated therewith provide non-volatile storage of the data, the data structure, the computer executable instruction, and others. In the case of the computer 1102, the drives and the media correspond to storing of predetermined data in an appropriate digital format. In the description of the computer readable media, the mobile optical media such as the HDD, the mobile magnetic disk, and the CD or the DVD are mentioned, but it will be well appreciated by those skilled in the art that other types of media readable by the computer such as a zip drive, a magnetic cassette, a flash memory card, a cartridge, and others may also be used in an exemplary operating environment and further, the predetermined media may include computer executable commands for executing the methods of the present disclosure. Multiple program modules including an operating system 1130, one or more application programs 1132, other program module 1134, and program data 1136 may be stored in the drive and the RAM 1112. All or some of the operating system, the application, the module, and/or the data may also be cached in the RAM 1112. It will be well appreciated that the present disclosure may be implemented in operating systems which are commercially usable or a combination of the operating systems.

A user may input instructions and information in the computer 1102 through one or more wired/wireless input devices, for example, pointing devices such as a keyboard 1138 and a mouse 1140. Other input devices (not illustrated) may include a microphone, an IR remote controller, a joystick, a game pad, a stylus pen, a touch screen, and others. These and other input devices are often connected to the processing device 1104 through an input device interface 1142 connected to the system bus 1108, but may be connected by other interfaces including a parallel port, an IEEE 1394 serial port, a game port, a USB port, an IR interface, and others.

A monitor 1144 or other types of display devices are also connected to the system bus 1108 through interfaces such as a video adapter 1146, and the like. In addition to the monitor 1144, the computer generally includes other peripheral output devices (not illustrated) such as a speaker, a printer, others.

The computer 1102 may operate in a networked environment by using a logical connection to one or more remote computers including remote computer(s) 1148 through wired and/or wireless communication. The remote computer(s) 1148 may be a workstation, a computing device computer, a router, a personal computer, a portable computer, a micro-processor based entertainment apparatus, a peer device, or other general network nodes and generally includes multiple components or all of the components described with respect to the computer 1102, but only a memory storage device 1150 is illustrated for brief description. The illustrated logical connection includes a wired/wireless connection to a local area network (LAN) 1152 and/or a larger network, for example, a wide area network (WAN) 1154. The LAN and WAN networking environments are general environments in offices and companies and facilitate an enterprise-wide computer network such as Intranet, and all of them may be connected to a worldwide computer network, for example, the Internet.

When the computer 1102 is used in the LAN networking environment, the computer 1102 is connected to a local network 1152 through a wired and/or wireless communication network interface or an adapter 1156. The adapter 1156 may facilitate the wired or wireless communication to the LAN 1152 and the LAN 1152 also includes a wireless access point installed therein in order to communicate with the wireless adapter 1156. When the computer 1102 is used in the WAN networking environment, the computer 1102 may include a modem 1158 or has other means that configure communication through the WAN 1154 such as connection to a communication computing device on the WAN 1154 or connection through the Internet. The modem 1158 which may be an internal or external and wired or wireless device is connected to the system bus 1108 through the serial port interface 1142. In the networked environment, the program modules described with respect to the computer 1102 or some thereof may be stored in the remote memory/storage device 1150. It will be well known that an illustrated network connection is exemplary and other means configuring a communication link among computers may be used.

The computer 1102 performs an operation of communicating with predetermined wireless devices or entities which are disposed and operated by the wireless communication, for example, the printer, a scanner, a desktop and/or a portable computer, a portable data assistant (PDA), a communication satellite, predetermined equipment or place associated with a wireless detectable tag, and a telephone. This at least includes wireless fidelity (Wi-Fi) and Bluetooth wireless technology. Accordingly, communication may be a predefined structure like the network in the related art or just ad hoc communication between at least two devices.

The wireless fidelity (Wi-Fi) enables connection to the Internet, and the like without a wired cable. The Wi-Fi is a wireless technology such as the device, for example, a cellular phone which enables the computer to transmit and receive data indoors or outdoors, that is, anywhere in a communication range of a base station. The Wi-Fi network uses a wireless technology called IEEE 802.11(a, b, g, and others) in order to provide safe, reliable, and high-speed wireless connection. The Wi-Fi may be used to connect the computers to each other or the Internet and the wired network (using IEEE 802.3 or Ethernet). The Wi-Fi network may operate, for example, at a data rate of 11 Mbps (802.11a) or 54 Mbps (802.11b) in unlicensed 2.4 and 5GHz wireless bands or operate in a product including both bands (dual bands).

It will be appreciated by those skilled in the art that information and signals may be expressed by using various different predetermined technologies and techniques. For example, data, instructions, commands, information, signals, bits, symbols, and chips which may be referred in the above description may be expressed by voltages, currents, electromagnetic waves, magnetic fields or particles, optical fields or particles, or predetermined combinations thereof.

It may be appreciated by those skilled in the art that various exemplary logical blocks, modules, processors, means, circuits, and algorithm steps described in association with the exemplary embodiments disclosed herein may be implemented by electronic hardware, various types of programs or design codes (for easy description, herein, designated as software), or a combination of all of them. In order to clearly describe the intercompatibility of the hardware and the software, various exemplary components, blocks, modules, circuits, and steps have been generally described above in association with functions thereof. Whether the functions are implemented as the hardware or software depends on design restrictions given to a specific application and an entire system. Those skilled in the art of the present disclosure may implement functions described by various methods with respect to each specific application, but it should not be interpreted that the implementation determination departs from the scope of the present disclosure.

Various exemplary embodiments presented herein may be implemented as manufactured articles using a method, a device, or a standard programming and/or engineering technique. The term manufactured article includes a computer program, a carrier, or a medium which is accessible by a predetermined computer-readable storage device. For example, a computer-readable storage medium includes a magnetic storage device (for example, a hard disk, a floppy disk, a magnetic strip, or the like), an optical disk (for example, a CD, a DVD, or the like), a smart card, and a flash memory device (for example, an EEPROM, a card, a stick, a key drive, or the like), but is not limited thereto. Further, various storage media presented herein include one or more devices and/or other machine-readable media for storing information.
It will be appreciated that a specific order or a hierarchical structure of steps in the presented processes is one example of exemplary accesses. It will be appreciated that the specific order or the hierarchical structure of the steps in the processes within the scope of the present disclosure may be rearranged based on design priorities. Appended method claims provide elements of various steps in a sample order, but the method claims are not limited to the presented specific order or hierarchical structure.

The description of the presented exemplary embodiments is provided so that those skilled in the art of the present disclosure use or implement the present disclosure. Various modifications of the exemplary embodiments will be apparent to those skilled in the art and general principles defined herein can be applied to other exemplary embodiments without departing from the scope of the present disclosure. Therefore, the present disclosure is not limited to the exemplary embodiments presented herein, but should be interpreted within the widest range which is coherent with the principles and new features presented herein.

## Claims

1. A method for generating disease prediction information using a biological signal performed by a computing device, the method comprising:
obtaining a biological signal at a time point of diagnosis;
obtaining a past biological signal at a past time point prior to the time point of diagnosis or information associated with the past biological signal; and
generating disease prediction information for the time point of diagnosis based on the biological signal at the time point of diagnosis, and the past biological signal at the past time point or the information associated with the past biological signal, using a neural network model.

2. The method of Claim 1, wherein the generating the disease prediction information for the time point of diagnosis comprises:
generating a first vector based on the biological signal;
generating a second vector based on the past biological signal or the information associated with the past biological signal; and
generating the disease prediction information for the time point of diagnosis based on the first vector and the second vector, using the neural network model.

3. The method of Claim 2, wherein the information associated with the past biological signal comprises at least one of:
time difference information between the past time point and the time point of diagnosis;
medical information at the past time point; or
reference prediction information generated for the past biological signal using a reference neural network model different from the neural network model.

4. The method of Claim 3, wherein the medical information at the past time point comprises:
medical measurement information measured within a predetermined time period from an acquisition time of the past biological signal.

5. The method of Claim 3, wherein the reference neural network model is a model configured to generate a reference prediction value based on a biological signal at a single time point.

6. The method of Claim 2, wherein the generating the disease prediction information for the time point of diagnosis based on the first vector and the second vector, using the neural network model comprises:
generating an integrated vector based on the first vector and the second vector; and
generating the disease prediction information for the time point of diagnosis based on the integrated vector, using the neural network model.

7. The method of Claim 6,
wherein the first vector and the second vector are vectors generated through different pre-processing methods,
wherein the first vector and the second vector comprise different numbers of elements, and
wherein the integrated vector comprises more elements of the first vector than elements of the second vector.

8. The method of Claim 2, wherein the obtaining the past biological signal at the past time point prior to the time point of diagnosis or the information associated with the past biological signal comprises:
obtaining a plurality of past biological signals for a plurality of past time points prior to the time point of diagnosis or information associated with the plurality of past biological signals, and
wherein the generating the second vector based on the past biological signal or the information associated with the past biological signal comprises: generating the second vector based on information associated with the plurality of past biological signals.

9. The method of Claim 8, wherein the information associated with the plurality of past biological signals comprises:
respective individual information associated with each past biological signal included in the plurality of past biological signals, and
wherein the respective individual information associated with each past biological signal comprises at least one of:
time difference information between the respective past time point and the time point of diagnosis;
medical information mapped to the respective past biological signal; or
reference prediction information generated for the respective past biological signal using a reference neural network model different from the neural network model.

10. The method of Claim 8, wherein the generating the second vector based on information associated with the plurality of past biological signals comprises:
generating supplementary biological signals in a number corresponding to a difference between the predetermined number and the number of the plurality of past biological signals, using at least one past biological signal among the plurality of past biological signals, when a number of the plurality of past biological signals is less than a predetermined number; and
generating the second vector based on the plurality of past biological signals and information associated with the supplementary biological signals.

11. The method of Claim 10, wherein the supplementary biological signals are generated based on a past biological signal of the earliest past time point among the plurality of past biological signals.

12. The method of Claim 8, wherein the information associated with the plurality of past biological signals comprises:
tabular data generated based on the plurality of past biological signals, and
wherein the tabular data comprises at least one type of data among:
a first type of data related to time difference;
a second type of data related to medical information;
a third type of data related to disease prediction based on a single time point; or
a fourth type of data related to missing information.

13. A structure of a neural network model implemented by a computing device, comprising:
a preprocessing module configured to preprocess a biological signal at a time point of diagnosis, and a past biological signal at a past time point prior to the time point of diagnosis or information associated with the past biological signal; and
a prediction module configured to generate disease prediction information based on data related to the biological signal generated by the preprocessing module and data related to the past biological signal generated by the preprocessing module.

14. The structure of the neural network model of Claim 13, wherein the preprocessing module comprises:
a first encoder module configured to generate a first vector based on the biological signal at the time point of diagnosis;
a second encoder module configured to generate a second vector based on the past biological signal or the information associated with the past biological signal; and
an integration module configured to generate an integrated vector based on the first vector and the second vector.

15. The structure of the neural network model of Claim 14,
wherein the prediction module is configured to generate the disease prediction information based on the integrated vector.

16. A computer program stored in a non-transitory computer-readable storage medium, wherein the computer program, when executed on one or more processors, causes the one or more processors to perform operations for generating disease prediction information using a biological signal, the operations comprising:
an operation of obtaining a biological signal at a time point of diagnosis;
an operation of obtaining a past biological signal at a past time point prior to the time point of diagnosis or information associated with the past biological signal; and
an operation of generating disease prediction information for the time point of diagnosis based on the biological signal at the time point of diagnosis, and the past biological signal at the past time point or the information associated with the past biological signal, using a neural network model.

17. A computing device comprising:
at least one processor; and
a memory,
wherein the at least one processor is configured to:
obtain a biological signal at a time point of diagnosis;
obtain a past biological signal at a past time point prior to the time point of diagnosis or information associated with the past biological signal; and
generate disease prediction information for the time point of diagnosis based on the biological signal at the time point of diagnosis, and the past biological signal at the past time point or the information associated with the past biological signal using a neural network model.
